# EUROPEAN PATENT SPECIFICATION

(11) **EP 2 025 283 B1**
(45) Date of publication and mention of the grant of the patent: **03.11.2010**
(21) Application number: 08014661.6
(22) Date of filing: 18.08.2008
(51) Int. Cl.: A61B 1/31, G02B 23/24, A61B 1/005

(54) **Device for insertion guide and endoscope having the same**
Vorrichtung für eine Einführungshilfe und Endoskop damit
Dispositif pour guide d'insertion et endoscope en disposant

(30) Priority: 16.08.2007 JP 2007212057
(43) Date of publication of application: 18.02.2009
(73) Proprietor: FUJIFILM Corporation, Minato-ku Tokyo (JP)
(72) Inventor: Yamamoto, Seiichi, Tokyo (JP)
(74) Representative: Klunker . Schmitt-Nilson . Hirsch

(56) References cited:
- WO-A-2006/121239
- GB-A- 2 437 339
- US-A- 4 207 872
- US-A1- 2004 030 454

## Description

### BACKGROUND OF THE INVENTION

### 1. Field of the Invention

The present invention relates to a device for insertion guide according to the preamble of claim 1, and endoscope having the same. More particularly, the present invention relates to a device for insertion guide and endoscope having the same, in which an insertion tube of the endoscope can be inserted easily and efficiently with force of propulsion even with a simple structure.

### 2. Description Related to the Prior Art

An endoscope such as a colonoscope is known in the field of medical instruments. An insertion tube is inserted in a small intestine, large intestine or the like in a gastrointestinal tract of a patient's body. An intestinal wall is reached through a tortuous path, and observed with the endoscope to diagnosis and treatment. A head portion or video imaging device or probe at a distal end is positioned on the insertion tube, and has an image pickup unit which creates image data of an image. While an operator observes the image, a steering portion on the rear of the head portion is operated and bent to tilt the head portion. A direction of insertion of the head portion is changed to propel the insertion tube. There is a problem in that an image is difficult to recognize because of the tilt of the head portion during the image pickup. If an unskilled operator handles the endoscope, extremely long time may be taken for exact insertion, because he or she may miss the insertion direction. Various techniques have been developed for facilitating the handling of the insertion tube in the insertion.

U.S.P. No. 6,071,234 (corresponding to JP-A 11-342106) discloses the use of an endless belt extending in the axial direction of the insertion tube. Force of propulsion is exerted by driving the endless belt. U.S.P. No. 2008/009675 (corresponding to JP-A 2006-230620) discloses a tube in a spiral shape for propulsion. The tube is rotated to create force of propulsion. U.S.P. No. 6,988,986 (corresponding to JP-A 2005-534367) discloses a loop disposed on the peripheral surface of the insertion tube or carrier. The loop is rotated about its axis to create force of propulsion of the insertion tube relative to the intestinal wall. JP-A 2004-209271 discloses a vibrator disposed near to the steering portion for reducing friction between the steering portion and the intestinal wall. U.S.P. No. 5,482,029 (corresponding to JP-B 3378298) discloses a structure of plural segments arranged in the axial direction to constitute the insertion tube. The segments are constructed with different flexibility between those. According to one of body parts in the passage in a body cavity, the flexibility of the segments is changed.

JP-U 5-043114 discloses a structure including a resilient mechanism, the head portion and a proximal end portion. The resilient mechanism expands and contracts in the axial direction. The head portion and the proximal end portion are positioned at ends of the resilient mechanism. A suction pad is associated with each of the head portion and the proximal end.portion, and is controlled for the suction. The insertion tube is moved and propelled by controlling the expansion and contraction of the resilient mechanism.

Also, JP-A 8-019618 discloses a structure having a portion with shape memory alloy for bending the steering portion of the insertion tube. JP-A 2004-041700 discloses a type of the endoscope including the insertion tube for viewing, an insertion tube for lighting, and an insertion tube with a channel. A double balloon mechanism is associated with each of the three of the insertion tubes for self propulsion. The insertion tubes are positioned with regularized ends in the entry to the body, and operated for observation and treatment. Their insertion is facilitated by reducing the diameter of each of the insertion tubes.

However, it is supposed that no sufficient force of propulsion is available according to the techniques of U.S.P. No. 6,071,234 (corresponding to JP-A 11-342106), U.S.P. No. 2008/009675 (corresponding to JP-A 2006-230620), and U.S.P. No. 6, 988, 986 (corresponding to JP-A 2005-534367), because a slip is likely to occur between the intestinal wall and the insertion tube (with the endless belt, tube of propulsion, or the loop). Should the endoscope such as a colonoscope be constructed for high friction according to those documents, the intestinal wall is very likely to be wounded. Also, it is necessary according to those documents to modify the form of the endoscope. There is no idea of utilizing an available type of the endoscope in a known form.

In JP-A 2004-209271, the friction is reduced by the vibrator. In U.S.P. No. 5,482,029 (corresponding to JP-B 3378298), the bendable property of the insertion tube is changed over according to body parts of interest for insertion. However, those documents do not disclose exertion of force of propulsion of the insertion tube. JP-U 5-043114, the suction with the suction pads and the expansion and contraction of the resilient mechanism must be repeated. Considerable time is required for the reach to an object of interest. In JP-A 8-019618, the portion with shape memory alloy is used for bending the steering portion. However, the insertion direction of the insertion tube may be missed because the head portion or video imaging device or probe at a distal end is swung. In JP-A 2004-041700, the propulsion force with the double-balloon structure may require long time until the reach to an object of interest. This structure also requires a complicated construction of the endoscope.

In accordance with the preamble of claim 1, US-A-4 207 872 discloses a device for insertion guide, in which the propulsion fin portion comprises a plurality of hollow resilient protrusions integral with a resilient outer wall of the base tube. The driving mechanism comprises a fluid pumping assembly which is used to establish a pressure within the hollow protrusions. Due to such pressure, the protrusions project outwardly and rearwardly, thereby propelling the head portion.

### SUMMARY OF THE INVENTION

In view of the foregoing problems, an object of the present invention is to provide a device for insertion guide and endoscope having the same, in which an insertion tube of the endoscope can be inserted easily and efficiently with force of propulsion even with a simple structure.

In order to achieve the above and other objects and advantages of this invention, a device for insertion guide includes the features of claim 1.

The driving mechanism includes a balloon secured between the head portion and the propulsion fin portion. A fluid pumping assembly causes fluid to flow into and out of the balloon, to shift the propulsion fin portion by expanding and contracting the balloon.

In a preferred embodiment, the driving mechanism includes an actuator, constituted by a shape memory material, and secured to the propulsion fin portion. A drive control unit powers the actuator to shift the propulsion fin portion.

The actuator includes a coil, formed from the shape memory material, shiftable by control of the powering, for expanding to set the propulsion fin portion in the closed position, and for contracting to set the propulsion fin portion in the open position.

The driving mechanism includes a pull line having front and rear ends, wherein the front end is secured to the distal fin end, the rear end is pulled to set the propulsion fin portion in the open position.

Furthermore, a passage channel is formed in the propulsion fin portion to extend between the distal fin end and a fin base thereof, has a predetermined thickness, and is shaped to open in the fin base, for passage of the pull line.

The driving mechanism further includes a winder, having the rear end of the pull line secured thereto, for rotating to unwind and wind the pull line, to shift the propulsion fin portion.

The propulsion fin portion is constituted by at least two propulsion fin portions.

The driving mechanism sets the propulsion fin portions in the open position in sequences different between the propulsion fin portions.

Furthermore, a mode selector is operable after propulsion with the propulsion fin portions, for setting a viewing mode. When the viewing mode is set, the driving mechanism keeps the at least two propulsion fin portions in the open position.

The head portion includes an imaging window for receiving image light of an object in the body. The at least two propulsion fin portions are opposed to one another so that the imaging window is located between.

The distal fin end is disposed in a field of view of the imaging window.

The driving mechanism sets the propulsion fin portions outside a field of view of the imaging window.

The driving mechanism sets the propulsion fin portions outside the field of view when the propulsion fin portions are in the open position.

The endoscope includes a lighting window formed in the head portion. A light source applies light to an object in the body through the lighting window. Furthermore, an anti-reflection surface is formed with the propulsion fin portion, opposed to the head portion, for preventing reflection of the light.

The endoscope includes a lighting window formed in the head portion. A light source applies light to an object in the body through the lighting window. Furthermore, a reflection surface is formed with the propulsion fin portion, for reflecting the light from the lighting window toward the object.

In a preferred embodiment, the endoscope is a colonoscope.

Also, an endoscope is provided, including an insertion guide device according to claim 1.

Consequently, an insertion tube of the endoscope can be inserted easily and efficiently with force of propulsion even with a simple structure, because the propulsion fin portion facilitates advance of the head portion of the endoscope.

### BRIEF DESCRIPTION OF THE DRAWINGS

The above objects and advantages of the present invention will become more apparent from the following detailed description when read in connection with the accompanying drawings, in which:
Fig. 1 is a plan illustrating a colonoscopic system;
Fig. 2 is a horizontal section, illustrating a device for insertion guide and an insertion tube;
Fig. 3 is a front elevation illustrating a front surface of the device for insertion guide and insertion tube;
Fig. 4 is a horizontal section, illustrating a state of the colonoscope in the propulsion;
Fig. 5A is an explanatory view in section, illustrating another preferred embodiment in which shape memory alloy is used for shifting a propulsion fin;
Fig. 5B is an explanatory view in section, illustrating the same as Fig. 5A but in an open position;
Fig. 6A is an explanatory view in section, illustrating still another preferred embodiment in which a pull line of wire is used for shifting a propulsion fin;
Fig. 6B is an explanatory view in section, illustrating the same as Fig. 6A but in an open position;
Fig. 7 is an explanatory view in section, illustrating a construction having a motor for winding the pull line of wire; and
Fig. 8 is an explanatory view in section, illustrating in which a reflection surface is formed on a propulsion fin.

### DETAILED DESCRIPTION OF THE PREFERRED EMBODIMENT(S) OF THE PRESENT INVENTION

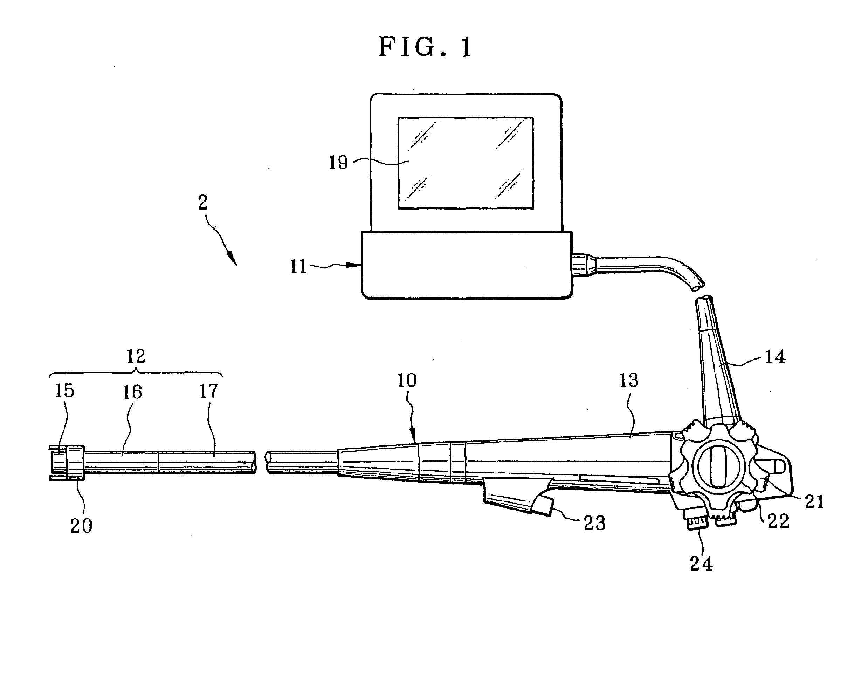

In Fig. 1, a colonoscopic system 2 as electronic endoscopic system includes a colonoscope 10 as electronic endoscope, a video processor 11 and a light source. The colonoscope 10 includes an insertion tube 12, a handle 13, and a cable 14. The handle 13 is a base from which the insertion tube 12 extends. The cable 14 extends for connection with the light source. A device for insertion guide 20 is attached to the colonoscope 10 for introducing access to a gastrointestinal tract of a patient's body in the medical diagnosis.

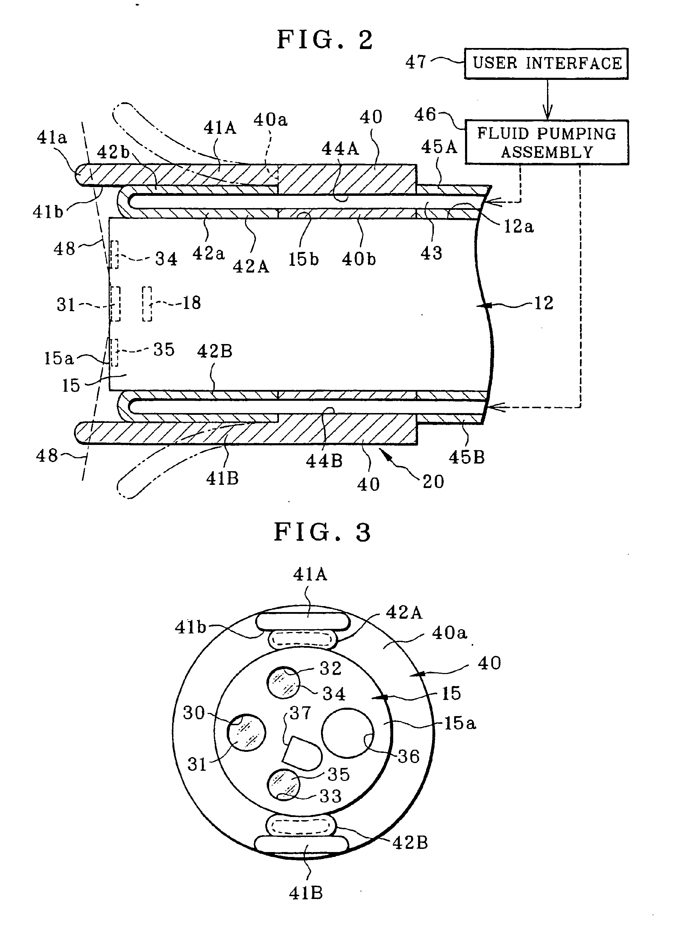

The insertion tube 12 includes a head portion 15 or video imaging device or probe at a distal end, a steering portion 16, and a flexible portion 17. The head portion 15 has a rigid body. In Fig. 2, an image pickup unit 18 or CCD image sensor is incorporated in the head portion 15 for image pickup of an object of interest in the gastrointestinal tract. Image data of the object is created by the image pickup unit 18, and is transmitted to the video processor 11 with a connection line passed through the insertion tube 12, the handle 13 and the cable 14. The video processor 11 processes the image data in image processing. A monitor display panel 19 is caused to display a medical image. Also, a light guide fiber is passed through the insertion tube 12, the handle 13 and the cable 14, and guides light from the light source toward the head portion 15.

A vertical steering wheel 21 is disposed on the handle 13, and rotated to bend the steering portion 16 up and down in a curved shape. A horizontal steering wheel 22 on the handle 13 is rotated to bend the steering portion 16 to the right and left in a curved shape. The vertical and horizontal steering wheels 21 and 22 are rotated to tilt the steering portion 16 to direct the head portion 15 in directions according to user preference in the body.

A first forceps opening 23 is formed in the handle 13 for insertion of a forceps or treatment device. A forceps channel is formed through the insertion tube 12 and extends from the first forceps opening 23. An air/water supply button 24 is disposed on the handle 13, and depressible for selective supply of air and water through a supply channel in the insertion tube 12.

In Figs. 2 and 3, the head portion 15 has a front end surface 15a or imaging device surface. An imaging window 30 is disposed in the front end surface 15a. An objective lens 31 is mounted in the imaging window 30, and focuses image light of an object in the body on the image pickup unit 18. Two lighting windows 32 and 33 are disposed on the front end surface 15a so that the imaging window 30 is positioned between those. Light source lenses 34 and 35 are mounted in respectively the lighting windows 32 and 33. Light emitted by a light source 38 is applied to the object in the body through the light guide fiber and the light source lenses 34 and 35. Also, a second forceps opening 36 and a nozzle 37 are formed in the front end surface 15a. The second forceps opening 36 communicates with the forceps channel. The nozzle 37 is open in a horizontal direction toward the imaging window 30. The nozzle 37 communicates with the supply channel, and ejects air and water selectively.

The device for insertion guide 20 is fitted on the head portion 15 for assistance to the insertion tube 12 toward the gastrointestinal tract. The device for insertion guide 20 can be attached to a colonoscope of a widely available type. The device for insertion guide 20 includes a base tube 40, propulsion fins 41A and 41B, and balloons 42A and 42B. The base tube 40 is attached to the head portion 15. The propulsion fins 41A and 41B protrude from a front surface 40a of the base tube 40 in an axial direction. The balloon 42A is disposed between the propulsion fin 41A and a peripheral surface 15b of the head portion 15. The balloon 42B is disposed between the propulsion fin 41B and the peripheral surface 15b.

The propulsion fins 41A and 41B are formed on the base tube 40 as one piece. Note that the propulsion fins 41A and 41B may be prepared separately, and attached to the base tube 40 in the assembly of the device for insertion guide 20. An inner diameter of the base tube 40 is substantially equal to an outer diameter of the head portion 15. An inner surface 40b of the base tube 40 is attached to the peripheral surface 15b of the head portion 15 by adhesion. The imaging window 30 is disposed between the propulsion fins 41A and 41B. The propulsion fin 41A is disposed on an outer side of the lighting window 32. The propulsion fin 41B is disposed on an outer side of the lighting window 33.

Examples of materials of the propulsion fin 41A are rubber, resin and the like. A distal fin end 41a of the propulsion fin 41A in a quadrilateral shape is formed with a curvature so as not to pierce or wound an intestinal wall. The propulsion fin 41A is movable between a closed position and an open position of the phantom line. When the propulsion fin 41A is in the closed position, the distal fin end 41a is near to the peripheral surface 15b of the head portion 15 as indicated by the solid line. When the propulsion fin 41A is in the open position, the distal fin end 41a comes away from the peripheral surface 15b. The distal fin end 41a is also shifted toward the proximal side of the handle 13 in the axial direction of the head portion 15 in comparison with the closed position. The propulsion fin 41A extends along the head portion 15 in the closed position, and is deployed in the open position with a curvature.

A field of view 48 is indicated by the phantom line, and is a region of an object which can be imaged with the image pickup unit 18 through the imaging window 30. When the propulsion fin 41A is in the closed position, the distal fin end 41a is positioned in the field of view 48. When the propulsion fin 41A is in the open position, the distal fin end 41a is positioned outside the field of view 48. The distal fin end 41a is imaged when the propulsion fin 41A is in the closed position, but not when the propulsion fin 41A is in the open position. An end retracting structure is constituted, and sets the outside of the field of view 48 at the time of the open position of the propulsion fin 41A.

An inner surface 41b of the distal fin end 41a of the propulsion fin 41A is finished with an anti-reflection surface. The anti-reflection surface prevents entry of light to the objective lens 31 with reflection on the inner surface 41b after emission from the lighting window 32. This is effective in keeping the easily viewable property of the image. Examples of types of the anti-reflection surface include a black layer of coating applied on the inner surface 41b, a mat surface without gloss, and the like.

The balloon 42A is expandable, and includes a proximal region 42a and an inflatable region 42b. The proximal region 42a is attached to the peripheral surface 15b of the head portion 15 by adhesion. The inflatable region 42b is set on the propulsion fin 41A. Fluid 43, such as water, air or the like is contained in the balloon 42A. The balloon 42A expands when the fluid 43 flows in, and contracts when the fluid 43 flows out. The propulsion fin 41A comes to the open position when the balloon 42A expands, and comes to the closed position when the balloon 42A contracts.

A conduit 44A is formed through the base tube 40 to supply the fluid 43. An inlet of the balloon 42A is connected with a distal end of the conduit 44A. Also, a pipe 45A is positioned to extend on an outer surface 12a of the insertion tube 12, and supplies the fluid 43. A first end of the pipe 45A is connected with a proximal end of the conduit 44A. A second end of the pipe 45A is connected with a fluid pumping assembly 46 or dispenser. The pipe 45A is formed from a material which does not expand or contract even in a flow of the fluid 43.

The propulsion fin 41B is constructed equally to the propulsion fin 41A. The balloon 42B is constructed equally to the balloon 42A. A conduit 44B structurally the same as the conduit 44A is connected with the balloon 42B. A pipe 45B structurally the same as the pipe 45A is connected with the conduit 44B. The fluid 43 is caused to flow by the fluid pumping assembly 46, the conduits 44A and 44B and the pipes 45A and 45B.

The fluid pumping assembly 46 is so constructed to cause the fluid 43 to flow to and from the pipes 45A and 45B in a manner discrete from one another. The fluid pumping assembly 46 is operable selectively in an insertion mode and a viewing mode. In the insertion mode, the fluid pumping assembly 46 causes the balloons 42A and 42B to expand alternately one after another. In the viewing mode, the balloons 42A and 42B continue the expanded state. A user interface 47 is operable to change over the modes of the fluid pumping assembly 46 and turn on and off the power source of the fluid pumping assembly 46. The user interface 47 is externally attached to the handle 13 of the colonoscope 10. Note that the fluid pumping assembly 46 may be initially separate from and mounted on the colonoscope 10, but can be incorporated in the colonoscope 10 as a structure of a small size. An example of the fluid pumping assembly 46 is constituted by two small pumps.

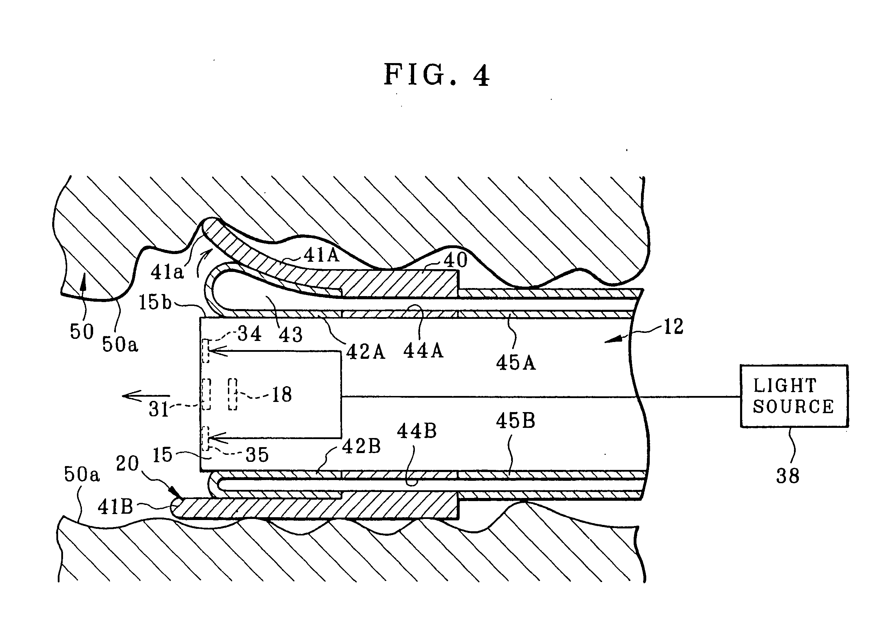

The operation of the embodiment is described by referring to Fig. 4. A large intestine 50 or colon of the patient is inspected by use of the colonoscope 10. At first, the light source 38 is turned on. The head portion 15 of the insertion tube 12 is inserted in the large intestine 50 through the anus. While an intestinal wall 50a is illuminated, an image is picked up by the image pickup unit 18 and displayed on the display panel 19 for observation. In the inspection of the large intestine 50, the propulsion fins 41A and 41B are set to contact the intestinal wall 50a or near to the intestinal wall 50a, as aspiration is effected to reduce a diameter of space in the large intestine 50.

When the fluid pumping assembly 46 is driven in the insertion mode by operating the user interface 47, at first fluid is introduced into the balloon 42A as illustrated in Fig. 4. The balloon 42A expands to shift the propulsion fin 41A from the closed position to the open position. The propulsion fin 41A pushes the intestinal wall 50a backwards in crawling movement, and thus comes to advance with respect from the intestinal wall 50a in an inward direction of the large intestine 50.

Then fluid is caused to flow out of the balloon 42A, which contracts to set the propulsion fin 41A back to the closed position. At the same time, the balloon 42B expands to shift the propulsion fin 41B from the closed position to the open position. The propulsion fin 41B swings to push back the intestinal wall 50a. Then the balloon 42B contracts to shift back the propulsion fin 41B to the closed position. Simultaneously, the balloon 42A expands to shift the propulsion fin 41A to the open position.

The above sequence is repeated, to set the propulsion fins 41A and 41B in the open position alternately one after another. Manual steering of the insertion tube 12 is assisted by the operation of the propulsion fins 41A and 41B to propel the head portion 15 of the insertion tube 12, which can reach a region having an object of interest in a short time easily. An operator inserts the insertion tube 12 by viewing an image on the display panel 19. Movement of the propulsion fins 41A and 41B can be viewed easily, as their ends are clearly displayed in the image on the display panel 19.

When the head portion 15 of the insertion tube 12 reaches a body part of interest, the user interface 47 is operated to set the viewing mode for the fluid pumping assembly 46. Then the propulsion fins 41A and 41B are both set in the open position, and come away from a region of the image on the display panel 19. Thus, the image can be observed safely without obstruction. After the inspection, the user interface 47 is operated to turn off the fluid pumping assembly 46 electrically. The propulsion fins 41A and 41B both come to the closed position. Then the insertion tube 12 is pulled and removed from the large intestine 50.

In the invention, the head portion 15 of the insertion tube 12 does not swing excessively in the course of insertion of the insertion tube 12. An insertion direction of the head portion 15 can be found easily. The insertion tube 12 can reach a body part of interest only in a short time even through a tortuous path owing to the movement of the propulsion fins 41A and 41B in contact with the intestinal wall.

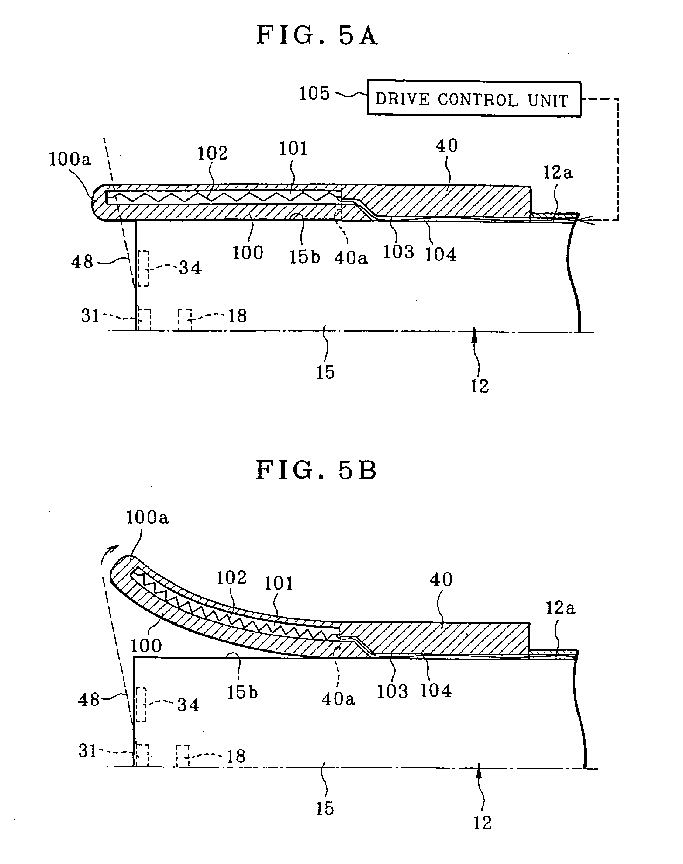

Another preferred embodiment is provided, in which a shape memory alloy is used in place of the balloon 42A for the propulsion fin 41A. In Fig. 5A, a propulsion fin 100 is disposed on the front surface 40a of the base tube 40 in place of each of the propulsion fins 41A and 41B.

A chamber 101 is formed in the propulsion fin 100, and positioned nearer to the outer side in the radial direction. An actuator with shape memory alloy 102 is contained in the chamber 101. A first end of the shape memory alloy 102 in a coil shape is connected with a distal fin end 100a of the propulsion fin 100. Its second end is attached to the front surface 40a of the base tube 40. The shape memory alloy 102 is in a contracted state in an environment of a predetermined temperature or higher.

A connection hole 103 is formed in the base tube 40. A connection line 104 is contained in the connection hole 103, and extends on an outer surface of the insertion tube 12. A first end of the connection line 104 is connected with the shape memory alloy 102. A second end of the connection line 104 is connected with a drive control unit 105 with a power source. The shape memory alloy 102 is powered by the drive control unit 105 through the connection line 104. Temperature of the shape memory alloy 102 increases at the time of powering.

In a room temperature, the shape memory alloy 102 is deformable with high degree of freedom. The propulsion fin 100 is in the closed position with the distal fin end 100a set on the front end surface 15a of the head portion 15. When the shape memory alloy 102 is energized by the drive control unit 105, the coil of the shape memory alloy 102 contracts as illustrated in Fig. 5B, and becomes curved to make the propulsion fin 100 convex in a downward direction. The propulsion fin 100 comes to the open position with the distal fin end 100a away from the head portion 15. When powering of the shape memory alloy 102 discontinues, its temperature becomes lower than a predetermined level, to set the shape memory alloy 102 back to the closed position. Powering of the drive control unit 105 is repeated, to shift the propulsion fin 100 between the open and closed positions, to propel the insertion tube 12 effectively.

Note that it is preferable to drive two propulsion fins 100 alternately in the base tube 40, to finish the inner surface of the end of the propulsion fins 100 with an anti-reflection surface, and to offset the propulsion fins 100 from the field of view of the image pickup unit 18 at the time of the open position, in the manner the same as the first embodiment.

In the embodiment, the shape memory alloy 102 is in a coil shape to shift the propulsion fin 100. Furthermore, the shape memory alloy 102 of a line shape may be used as an actuator. In a condition of a predetermined temperature or higher, the shape memory alloy 102 becomes bent to shift the propulsion fin 100 to the open position.

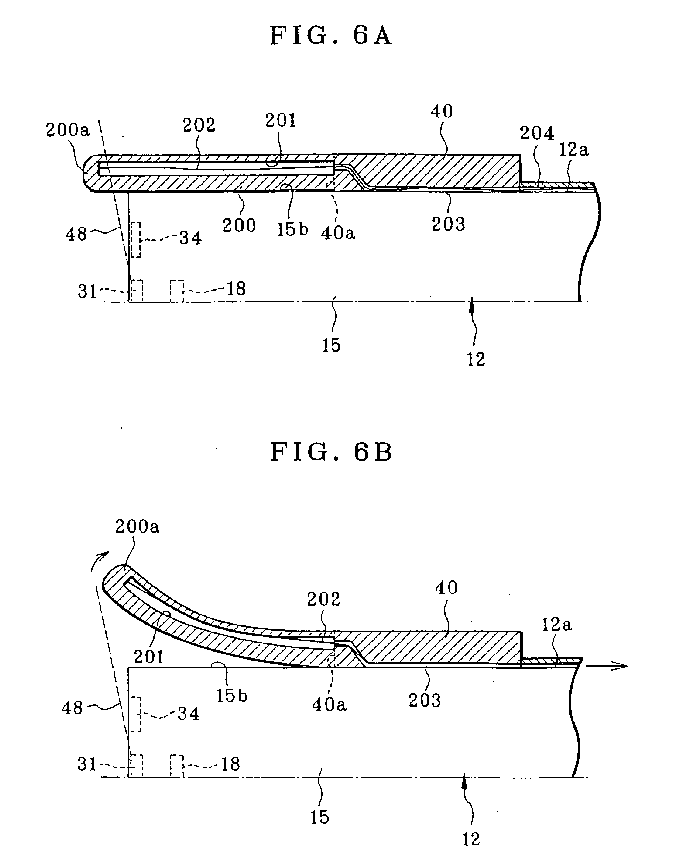

A still another preferred embodiment is described now, in which a pull line of wire is used in place of the balloon 42A. In Fig. 6A, a propulsion fin 200 is disposed on the front surface 40a of the base tube 40 in place of each of the propulsion fins 41A and 41B.

A passage channel 201 is formed in the propulsion fin 200, and positioned nearer to an outer side with respect to a radial direction. A pull line of wire 202 extends through the passage channel 201. A distal fin end 200a of the propulsion fin 200 is connected with a front end of the pull line 202.

A connection channel 203 is formed in the base tube 40. A middle portion of the pull line 202 is contained in the connection channel 203. Furthermore, a connection channel 204 is formed in a peripheral portion of the insertion tube 12. The pull line 202 extends through the connection channel 204 toward the handle 13. The rear end of the pull line 202 protrudes from an exit (not shown) formed in the handle 13, and can be pulled manually.

When the pull line 202 is not pulled, the distal fin end 200a is set in the closed position on the peripheral surface 15b of the head portion 15 by the resiliency of the propulsion fin 200. When the pull line 202 is pulled, the propulsion fin 200 becomes curved in a downwards convex manner as illustrated in Fig. 6B. The distal fin end 200a of the propulsion fin 200 comes to the open position away from the head portion 15. When the pull line 202 is released manually, the propulsion fin 200 is set again to the closed position by its resiliency. The pull of the pull line 202 is repeated to propel the insertion tube 12, as the propulsion fin 200 is shifted between the open and closed positions repeatedly.

Note that it is preferable to drive two propulsion fins 200 alternately in the base tube 40, to finish the inner surface of the end of the propulsion fins 200 with an anti-reflection surface, and to offset the propulsion fins 200 from the field of view of the image pickup unit 18 at the time of the open position, in the manner the same as the first embodiment.

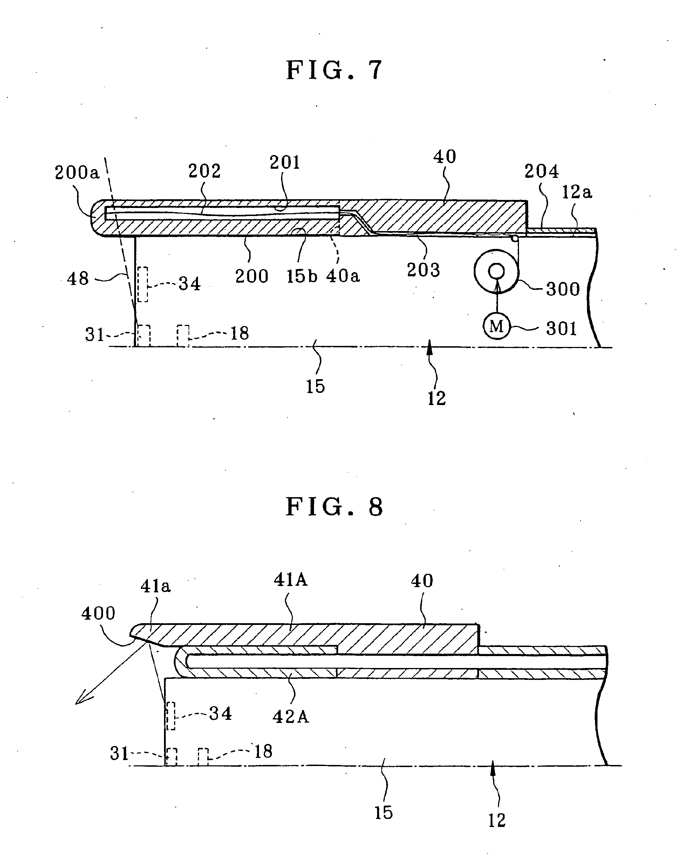

In the above embodiment, the rear end of the pull line 202 is pulled manually. In Fig. 7, another preferred embodiment is illustrated, in which a rotatable spindle 300 of a winder is connected with the rear end of the pull line 202. A motor 301 rotates the spindle 300 to pull the pull line 202. Examples of the motor 301 may be an ultrasonic motor, MEMS motor, and other motors of a micro type. The motor 301 can be preferably contained in the head portion 15, the handle 13 or the like of the colonoscope 10.

In Fig. 8, a preferred embodiment with a reflection surface 400 is illustrated. The reflection surface 400 is formed on the distal fin end 41a of the propulsion fin 41A, and reflects light from the light source lens 34 to an object in the body. This is in place of the anti-reflection surface of the first embodiment. The reflection surface 400 is effective in raising the brightness of the image for easy observation.

Note that the pipe 45A is disposed outside the insertion tube 12 in the above embodiment, but may be disposed within the insertion tube 12. Also, the connection line 104 and the pull line 202 of the various embodiments can be positioned to extend through a channel or hole in the insertion tube 12.

In the embodiments, the two propulsion fins are used. However, only one propulsion fin or three or more may be used. When a plurality of the propulsion fins are used, it is preferable to dispose the propulsion fins so that the imaging window 30 is positioned as a center between those.

In the above embodiments, the two propulsion fins are alternately driven. However, the propulsion fins may be driven simultaneously. In other words, the propulsion fins can be in the closed position at the same time. After this, the propulsion fins can be in the open position at the same time.

In the above embodiments, the propulsion fins are positioned in the field of view when in the closed position, and positioned in a region offset from the field of view when in the open position. However, the propulsion fins can be constructed to be set always in the field of view irrespective of the open and closed positions. Also, the propulsion fins can be slidable in the axial direction of the insertion tube. If required, the propulsion fins can be moved back and offset from the field of view.

In the above embodiments, bendable materials are used for the propulsion fin to have a bendable property. However, a propulsion fin may be formed from a rigid material without a bendable property, and may be supported on the base tube 40 in a pivotally rotatable manner between the open and closed positions. It is possible to bias the propulsion fin in the closed position, and to shift the propulsion fin to the open position by utilizing a pull line of wire, motor and other driving unit.

In the above embodiments, the endoscope is the colonoscope. However, an endoscope of the invention may be other types, such as an endoscope of an ocular type in which an image guide fiber is used to transmit image light. In the above embodiments, the endoscope is for medical use. However, the endoscope of the invention may be a fiberscope, borescope or other optical instruments for industrial use, such as a type for inspecting piping.

In the above embodiments, the head portion has a CCD image sensor. However, a head portion in an endoscope of the invention can be an ultrasonic probe or other imaging device suitable in the field of diagnosis.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A device for insertion guide, comprising:
a base tube (40) mounted on a head portion (15) of an insertion tube (12) of an endoscope (10);
a propulsion fin portion (41A, 41B, 100, 200) disposed on said base tube to extend in an axial direction of said head portion, and
a driving mechanism (42A, 42B, 102, 202) for shifting said propulsion fin portion (41A, 41B, 100, 200) between closed and open positions, **characterized in that** a distal fin end (41a, 100a, 200a) of said propulsion fin portion, when in the closed position, is disposed to extend along said head portion (15), and when in the open position, is deployed in a radial direction by setting away from said head portion (15) to propel said head portion (15) to be inserted in a body, wherein said head portion (15) includes an imaging window (30) for receiving image light of an object in said body; and
said distal fin end is disposed in a field of view (48) of said imaging window (30), when the propulsion fin portion is in the closed position.

2. A device for insertion guide as defined in claim 1, wherein said driving mechanism includes:
a balloon (42A, 42B) secured between said head portion and said propulsion fin portion;
a fluid pumping assembly (46) for causing fluid to flow into and out of said balloon, to shift said propulsion fin portion by expanding and contracting said balloon (42A, 42B).

3. A device for insertion guide as defined in claim 1, wherein said driving mechanism includes:
an actuator (102), constituted by a shape memory material, and secured to said propulsion fin portion;
a drive control unit (105) for powering said actuator to shift said propulsion fin portion.

4. A device for insertion guide as defined in claim 1, wherein said driving mechanism includes a pull line (202) having front and rear ends, wherein said front end is secured to said propulsion fin portion (200), said rear end is pulled to set said propulsion fin portion in said open position.

5. A device for insertion guide as defined in claim 4, wherein said driving mechanism further includes a motor (301) for unwinding and winding said rear end of said pull line.

6. A device for insertion guide as defined in claim 1, wherein said propulsion fin portion is constituted by at least two propulsion fin portions.

7. A device for insertion guide as defined in claim 6, wherein
said at least two propulsion fin portions are opposed to one another so that said imaging window is located between said at least two propulsion fin portions.

8. A device for insertion guide as defined in claim 6, wherein said driving mechanism sets said propulsion fin portions in said open position alternately with one another.

9. A device for insertion guide as defined in claim 8, further comprising a mode selector, operable after propulsion with said propulsion fin portions, for setting a viewing mode;
wherein when said viewing mode is set, said driving mechanism keeps said at least two propulsion fin portions in said open position.

10. A device for insertion guide as defined in claim 1,
said distal fin end is shiftable outside a field of view of said imaging window.

11. A device for insertion guide as defined in claim 10, wherein said distal fin end, when set in said open position by said driving mechanism, comes outside said field of view.

12. An endoscope comprising a device according to any of claims 1 to 11.

13. A device for insertion guide as defined in claim 1, wherein said endoscope includes:
a lighting window formed in said head portion;
a light source for applying light to an object in said body through said lighting window;
further comprising an anti-reflection surface, formed with said propulsion fin portion, opposed to said head portion, for preventing reflection of said light.

14. A device for insertion guide as defined in claim 1, wherein said endoscope includes:
a lighting window formed in said head portion;
a light source for applying light to an object in said body through said lighting window;
further comprising a reflection surface, formed with said propulsion fin portion, for reflecting said light from said lighting window toward said object.

15. A device for insertion guide as defined in claim 1, wherein said endoscope is a colonoscope.

16. An endoscope as defined in claim 12, wherein said endoscope is a colonoscope.

## Patentansprüche

1. Einführhilfenvorrichtung, umfassend:
einen Basistubus (40), der an einem Kopfteil (15) eines Einführschlauchs (12) eines Endoskops (10) angebracht ist;
einen Vortriebsfinnenabschnitt (41A, 41B, 100, 200), der an dem Basistubus angeordnet ist, um sich in axialer Richtung des Kopfteils zu erstrecken; und
einen Antriebsmechanismus (42A, 42B, 102, 202) zum Verschieben des Vortriebsfinnenabschnitts (41A, 41B, 100, 200) zwischen geschlossenen und geöffneten Stellungen, **dadurch gekennzeichnet, dass** ein distales Finnenende (41a, 100a, 200a) des Vortriebsfinnenabschnitts in dessen geschlossener Stellung so angeordnet ist, dass es sich entlang dem Kopfteil (15) erstreckt, während es in der geöffneten Stellung in radialer Richtung ausgefahren wird, indem es von dem Kopfteil (15) abgerückt wird, um den in einen Körper einzuführenden Kopfteil (15) vorzutreiben, wobei der Kopfteil (15) ein Abbildungsfenster (30) zum Aufnehmen von Bildlicht eines in dem Körper befindlichen Objekts enthält; und
das distale Finnenende in einem Gesichtsfeld (48) des Abbildungsfensters (30) angeordnet ist, wenn sich der Vortriebsfinnenabschnitt in der geschlossenen Stellung befindet

2. Vorrichtung nach Anspruch 1, bei der der Antriebsmechanismus enthält:
einen Ballon (42A, 42B), der zwischen dem Kopfteil und dem Vortriebsfinnenabschnitt befestigt ist;
eine Fluidpumpanordnung (46) zum Veranlassen von Fluid, in den und aus dem Ballon zu strömen, um den Vortriebsfinnenabschnitt durch Expandieren und Kontrahieren des Ballons (42A, 42B) zu verschieben.

3. Vorrichtung nach Anspruch 1, bei der der Antriebsmechanismus enthält:
einen Aktuator (102), gebildet durch einen Formspeicherwerkstoff und angebracht an dem Vortriebsfinnenabschnitt;
eine Antriebssteuereinheit (105) zum Speisen des Aktuators, um den Vortriebsfinnenabschnitt zu verschieben.

4. Vorrichtung nach Anspruch 1, bei dem der Antriebsmechanismus eine Zugleitung mit einem vorderen und einem hinteren Ende enthält, wobei das vordere Ende an dem Vortriebsfinnenabschnitt (2) befestigt ist und das hintere Ende gezogen wird, um den Vortriebsfinnenabschnitt in die geöffnete Stellung zu versetzen.

5. Vorrichtung nach Anspruch 4, bei der der Antriebsmechanismus außerdem einen Motor (301) zum Aufwickeln und Abwickeln des hinteren Endes der Zugleitung enthält.

6. Vorrichtung nach Anspruch 1, bei der der Vortriebsfinnenabschnitt durch mindestens zwei Vortriebsfinnenabschnitte gebildet wird.

7. Vorrichtung nach Anspruch 6, bei der die mindestens zwei Vortriebsfinnenabschnitte einander derart gegenüberliegen, dass sich das Abbildungsfenster zwischen den mindestens zwei Vortriebsfinnenabschnitten befindet.

8. Vorrichtung nach Anspruch 6, bei der der Antriebsmechanismus die Vortriebsfinnenabschnitte in der offenen Stellung abwechselnd zueinander versetzt.

9. Vorrichtung nach Anspruch 8, weiterhin umfassend einen Moduswähler, der nach Vortrieb mit Hilfe der Vortriebsfinnenabschnitte betreibbar ist, um einen Betrachtungsmodus einzustellen;
wobei, wenn der Betrachtungsmodus eingestellt ist, der Antriebsmechanismus die mindestens zwei Vortriebsfinnenabschnitte in der offenen Stellung hält.

10. Vorrichtung nach Anspruch 1, bei der das distale Finnenende aus einem Gesichtsfeld des Abbildungsfensters heraus verschiebbar ist.

11. Vorrichtung nach Anspruch 10, bei der das distale Finnenende bei Einstellung in die offene Stellung mit Hilfe des Antriebsmechanismus außerhalb des Gesichtsfeldes gelangt.

12. Endoskop mit einer Vorrichtung nach einem der Ansprüche 1 bis 11.

13. Vorrichtung nach Anspruch 1, bei der das Endoskop aufweist:
ein Beleuchtungsfenster, welches in dem Kopfteil ausgebildet ist;
eine Lichtquelle zum Aufbringen von Licht auf ein in dem Körper befindliches Objekt durch das Beleuchtungsfenster;
weiterhin umfassend eine Antireflexionsfläche, die zum Verhindern von Reflexion des Lichts bei dem Vortriebsfinnenabschnitt gegenüber dem Kopfteil ausgebildet ist.

14. Vorrichtung nach Anspruch 1, bei der das Endoskop enthält:
ein Beleuchtungsfenster, das in dem Kopfteil ausgebildet ist;
eine Lichtquelle zum Aufbringen von Licht auf ein in dem Körper befindliches Objekt durch das Beleuchtungsfenster;
weiterhin umfassend eine Reflexionsfläche, die an dem Vortriebsfinnenabschnitt ausgebildet ist, um aus dem Beleuchtungsfenster kommendes Licht in Richtung des Objekts zu reflektieren.

15. Vorrichtung nach Anspruch 1, bei dem das Endoskop ein Kolonoskop ist.

16. Endoskop nach Anspruch 12, bei dem das Endoskop ein Kolonoskop ist.

## Revendications

1. Dispositif pour guide d'insertion, comprenant :
un tube de base (40) monté sur une portion de tête (15) d'un tube d'insertion (12) d'un endoscope (10) ;
une portion en ailette de propulsion (41A, 41B, 100, 200) disposée sur ledit tube de base pour s'étendre dans une direction axiale de ladite portion de tête, et
un mécanisme d'entraînement (42A, 42B, 102, 202) pour faire passer ladite portion en ailette de propulsion (41A, 41B, 100, 200) entre une position fermée et une position ouverte, **caractérisé en ce qu'**une extrémité d'ailette distale (41a, 100a, 200a) de ladite portion en ailette de propulsion, lorsqu'elle est dans la position fermée, est disposée de manière à s'étendre le long de ladite portion de tête (15) et, lorsqu'elle est dans la position ouverte, est déployée dans une direction radiale en se dressant en éloignement de ladite portion de tête (15) pour propulser ladite portion de tête (15) pour l'introduire dans un corps, dans lequel ladite portion de tête (15) inclut une fenêtre d'imagerie (30) pour recevoir une lumière image d'un objet dans ledit corps ; et
ladite extrémité d'ailette distale est disposée dans un champ de vision (48) de ladite fenêtre d'imagerie (30), quand la portion en ailette de propulsion est dans la position fermée.

2. Dispositif pour guide d'insertion selon la revendication 1, dans lequel ledit mécanisme d'entraînement inclut :
un ballon (42A, 42B) fixé entre ladite portion de tête et ladite portion en ailette de propulsion ;
un ensemble formant pompe à fluide (46) pour amener du fluide à s'écouler en entrant et en sortant dudit ballon, pour déplacer ladite portion en ailette de propulsion par expansion et contraction dudit ballon (42A, 42B).

3. Dispositif pour guide d'insertion selon la revendication 1, dans lequel ledit mécanisme d'entraînement inclut :
un actionneur (102), constitué par un matériau à mémoire de forme, et attaché sur ladite portion en ailette de propulsion ;
une unité de commande d'entraînement (105) pour fournir une puissance audit actionneur pour déplacer ladite portion en ailette de propulsion.

4. Dispositif pour guide d'insertion selon la revendication 1, dans lequel ledit mécanisme d'entraînement inclut une ligne de traction (202) ayant une extrémité avant et une extrémité arrière, dans lequel ladite extrémité avant est fixée sur ladite portion en ailette de propulsion (200), et ladite extrémité arrière est tirée pour placer ladite portion en ailette de propulsion dans ladite position ouverte.

5. Dispositif pour guide d'insertion selon la revendication 4, dans lequel ledit mécanisme d'entraînement inclut encore un moteur (301) pour dérouler et pour enrouler ladite extrémité arrière de ladite ligne de traction.

6. Dispositif pour guide d'insertion selon la revendication 1, dans lequel ladite portion en ailette de propulsion est constituée par au moins deux portions en ailette de propulsion.

7. Dispositif pour guide d'insertion selon la revendication 6, dans lequel lesdites au moins deux portions en ailette de propulsion sont opposées l'une à l'autre de telle sorte que ladite fenêtre d'imagerie est placée entre lesdites au moins deux portions en ailette de propulsion.

8. Dispositif pour guide d'insertion selon la revendication 6, dans lequel ledit mécanisme d'entraînement établit lesdites portions en ailette de propulsion dans ladite position ouverte en alternance les unes par rapport aux autres.

9. Dispositif pour guide d'insertion selon la revendication 8, comprenant en outre un sélecteur de mode, capable de fonctionner après propulsion avec lesdites portions en ailette de propulsion, pour établir un mode de vision ;
dans lequel, quand ledit mode de vision est établi, ledit mécanisme d'entraînement maintient lesdites au moins deux portions en ailette de propulsion dans ladite position ouverte.

10. Dispositif pour guide d'insertion selon la revendication 1, dans lequel ladite extrémité d'ailette distale est capable de se déplacer à l'extérieur d'un champ de vision de ladite fenêtre d'imagerie.

11. Dispositif pour guide d'insertion selon la revendication 10, dans lequel ladite extrémité d'ailette distale, lorsqu'elle est placée dans ladite position ouverte par ledit mécanisme d'entraînement, vient à l'extérieur dudit champ de vision.

12. Endoscope comprenant un dispositif selon l'une quelconque des revendications 1 à 11.

13. Dispositif pour guide d'insertion selon la revendication 1, dans lequel ledit endoscope inclut :
une fenêtre d'éclairage formée dans ladite portion de tête ;
une source de lumière pour appliquer de la lumière à un objet dans ledit corps via ladite fenêtre d'éclairage ;
comprenant en outre une surface antireflets, formée avec ladite portion en ailette de propulsion, à l'opposé de ladite portion de tête, pour empêcher une réflexion de ladite lumière.

14. Dispositif pour guide d'insertion selon la revendication 1, dans lequel ledit endoscope inclut :
une fenêtre d'éclairage formée dans ladite portion de tête ;
une source de lumière pour appliquer de la lumière à un objet dans ledit corps via ladite fenêtre d'éclairage ;
comprenant en outre une surface de réflexion, formée avec ladite portion en ailette de propulsion, pour réfléchir ladite lumière depuis ladite fenêtre d'éclairage vers ledit objet.

15. Dispositif pour guide d'insertion selon la revendication 1, dans lequel ledit endoscope est un colonoscope.

16. Endoscope selon la revendication 12, dans lequel ledit endoscope est un colonoscope.
